(19) Europäisches Patentamt
European Patent Office
Office européen des brevets

(11) Veröffentlichungsnummer: **0 034 276**
**B1**

# (12) EUROPÄISCHE PATENTSCHRIFT

(45) Veröffentlichungstag der Patentschrift: **09.11.83**

(51) Int. Cl.³: **C 07 D 271/10**, A 61 K 31/495 // C07D405/12

(21) Anmeldenummer: **81100592.5**

(22) Anmeldetag: **28.01.81**

(54) **Phenylpiperazinderivate von 1,3,4-Oxadiazolylphenolen, ihre Herstellung und diese enthaltende therapeutische Mittel.**

(30) Priorität: **13.02.80 DE 3005287**

(43) Veröffentlichungstag der Anmeldung: **26.08.81 Patentblatt 81/34**

(45) Bekanntmachung des Hinweises auf die Patenterteilung: **09.11.83 Patentblatt 83/45**

(84) Benannte Vertragsstaaten: **AT BE CH DE FR GB IT LI LU NL SE**

(56) Entgegenhaltungen:
**DE - A - 2 811 638**
**GB - A - 1 164 572**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Thieme, Peter C. Dr., Dr.-Hans-Hoffmann-Strasse 5, D-6706 Wachenheim (DE)**
Erfinder: **Franke, Albrecht, Dr., Mandelring 11, D-6706 Wachenheim (DE)**
Erfinder: **Lenke, Dieter, Dr., Kekuleplatz 1, D-6700 Ludwigshafen (DE)**
Erfinder: **Lehmann, Hans Dieter, Dr., Im Hefen 15, D-6945 Hirschberg-Leutershauen (DE)**
Erfinder: **Gries, Josef, Dr., Roemerweg 43, D-6706 Wachenheim (DE)**

EP 0 034 276 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Phenylpiperazinderivate von 1,3,4-Oxadiazolylphenolen, ihre Herstellung und diese enthaltende therapeutische Mittel

Die vorliegende Erfindung betrifft neue Phenylpiperazinylpropanole von 1,3,4-Oxadiazolylphenolen und ihre physiologisch verträglichen Säureadditionssalze, Verfahren zu ihrer Herstellung und diese Verbindungen enthaltende pharmazeutische Zubereitungen, die bei der Behandlung der Hypertonie verwendet werden können.

Aus der DE-OS 28 11 638 sind Aminopropanolderivate von 1,3,4-Oxadiazolylphenolen bekannt, für die eine $\beta$-adrenolytische und blutdrucksenkende Wirkung beschrieben wird. Weiterhin ist beispielsweise das Arzneimittel Urapidil, ein Uracilderivat mit einem o-Methoxyphenyl-piperazinyl-Rest, als Antihypertonikum bekannt.

Es wurde gefunden, daß Verbindungen der allgemeinen Formel I,

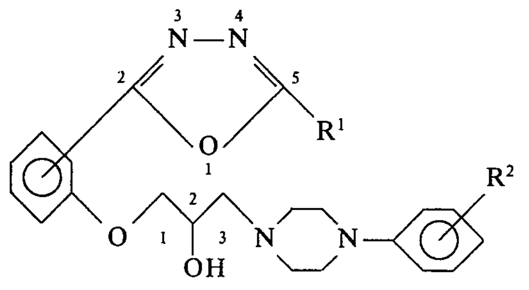

(I)

in denen $R^1$ ein Wasserstoffatom oder Methyl und $R^2$ ein Wasserstoffatom, ein Fluor- oder Chloratom, eine Methyl- oder eine Methoxy- oder Ethoxygruppe, wobei $R^2$ als Substituet des Phenylringes ein- oder zweifach vorhanden sein kann, bedeuten, und ihre Säureadditionssalze wertvolle pharmakologische Eigenschaften aufweisen.

Der 1,3,4-Oxadiazolyl-2-rest kann sich zu der Äthergruppe in o-, m- oder p-Stellung befinden, wovon die m-Stellung bevorzugt ist.

Der Substituent $R^2$ kann in o- m- oder p-Stellung zum Piperazinsubstituenten im Phenylring des Phenylpiperazin stehen.

Phenylreste mit zwei Substituenten $R^2$ sind beispielsweise o,o'-Dimethylphenyl oder o-p-Dimethoxyphenyl.

Dementsprechend sind als erfindungsgemäße Verbindungen der Formel I beispielsweise zu nennen:

1-[2-(1,3,4-Oxadiazolyl-2)-phenoxy]-3-[4-(2-methoxyphenyl)-piperazinyl-1]-propanol-2
1-[2-(5-Methyl-1,3,4-oxadiazolyl-2)-phenoxy]-3-[4-(2-methoxyphenyl)-piperazinyl-1]-propanol-2
1-[3-(1,3,4-Oxadiazolyl-2)-phenoxy]-3-[4-(2-methoxyphenyl)-piperazinyl-1]-propanol-2
1-[3-(1,3,4-Oxadiazolyl-2)-phenoxy]-3-[4-(2-äthoxyphenyl)-piperazinyl-1]-propanol-2
1-[3-(5-Methyl-1,3,4-oxadiazolyl-2)-phenoxy]-3-(4-phenylpiperazinyl-1)-propanol-2
1-[3-(5-Methyl-1,3,4-oxadiazolyl-2)-phenoxy]-3-[4-(2-methoxyphenyl)-piperazinyl-1]-propanol-2
1-[3-(5-Methyl-1,3,4-oxadiazolyl-2)-phenoxy]-3-[4-(2-äthoxyphenyl)-piperazinyl-1]-propanol-2
1-[3-(5-Methyl-1,3,4-oxadiazolyl-2)-phenoxy]-3-[4-(4-fluorphenyl)-piperazinyl-1]-propanol-2
1-[4-(1,3,4-Oxadiazolyl-2)-phenoxy]-3-[4-(4-fluorphenyl)-piperazinyl-1]-propanol-2
1-[4-(5-Methyl-1,3,4-oxadiazolyl-2)-phenoxy]-3-[4-(3-methoxyphenyl)-piperazinyl-1]-propanol-2.

Die erfindungsgemäßen Verbindungen werden hergestellt, indem man ein 1,3,4-Oxadiazolyl-2-phenyl-derivat der allgemeinen Formel II,

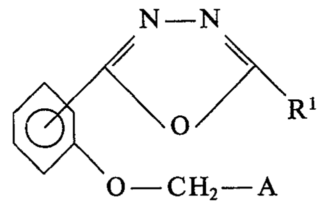

(II)

in der $R^1$ die für Formel I angegebenen Bedeutungen hat und in der A den Rest

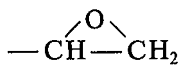 oder 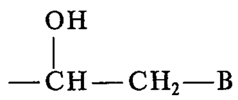

wobei B für eine nukleofuge Abgangsgruppe steht, bedeutet, mit einem Phenylpiperazin der allgemeinen Formel III

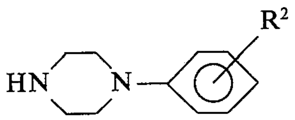

(III)

in der $R^2$ die für Formel I angegebenen Bedeutungen hat, zweckmäßigerweise in einem Lösungsmittel und gegebenenfalls in Gegenwart eines säurebindenden Mittels in an sich bekannter Weise umsetzt und die erhaltene Verbindung gegebenenfalls in das Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurerest in Betracht, wie der p-Toluolsulfonsäure-, der p-Brombenzolsulfonsäure- oder der Methansulfonsäurerest.

Die Umsetzungen werden bei Temperaturen von 10 bis 120°C, d. h. bei Raumtemperaturen oder bei höheren Temperaturen, zweckmäßig bei Temperaturen von 50 bis 120°C, durchgeführt. Die Umsetzungen können unter Atmosphärendruck oder in einem geschlossenen Gefäß unter erhöhtem Druck, gegebenenfalls unter Erwärmung auf den angegebenen Temperaturbereich, durchgeführt werden.

Die Ausgangsverbindungen können direkt, d. h. ohne Zugabe eines Verdünnungs- oder Lösungsmittels, umgesetzt werden.

Zweckmäßigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, von Dimethylsulfoxid oder in Gegenwart von Wasser oder in Mischungen der genannten Lösungsmittel, durchgeführt.

Bevorzugte Lösungsmittel bei der Umsetzung eines Epoxids der Formel II, beispielsweise von 2,3-Epoxypropoxyphenyl-1,3,4-oxadiazolen, mit einem Phenylpiperazin der Formel III sind niedere Alkohole, insbesondere Äthanol oder Isopropanol, wobei die Umsetzung bevorzugt bei Temperaturen von 50°C bis 120°C und bei Normaldruck durchgeführt wird. Bei der nukleophilen Substitution eines Restes B sind als Lösungsmittel ein niederes aliphatisches Keton, wie Aceton, Diäthylketon, Methylisopropylketon oder Methylisobutylketon, ein cyclischer gesättigter Äther, insbesondere Tetrahydrofuran oder Dioxan, oder ein Dialkylformamid, wie Dimethylformamid, und Temperaturen von 90 bis 120°C bevorzugt. Gegebenenfalls wird die Reaktion in Gegenwart einer katalytischen Menge Natrium- oder Kaliumjodid durchgeführt.

Es sei erwähnt, daß als Ausgangsverbindung der Formel II gegebenenfalls auch eine Mischung des Epoxids mit einem Halogenhydrin in Betracht kommt, da bei der technischen Herstellung der Ausgangsverbindungen der Formel II derartige Gemische unter Umständen entstehen können.

Bei einer zweckmäßigen Ausführungsform zur nukleophilen Substitution des Restes B durch das verwendete Phenylpiperazinderivat wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin wie Pyridin oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuß verwendet.

Gegebenenfalls ist es zweckmäßig, das zur Umsetzung verwendete Phenylpiperazinderivat in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, überführen in eine Säureadditionsverbindung oder durch Säulenchromatographie.

Die Ausgangsverbindungen der Formel (II) können durch Alkylierung der 1,3,4-Oxadiazolylphenole, die nach Literaturvorschrift hergestellt werden können (J. Maillard, M. Vincent, V. Van-Tri- Bulletin de la Societe Chimique de France 1966, S. 376 ff), mit einem Epihalogenhydrin oder einem $\alpha,\omega$-Dihalogen-2-propanol erhalten werden.

Als Epihalogenhydrine kommen Epichlorhydrin, Epibromhydrin und Epijodhydrin und als $\alpha,\omega$-Dihalogen-2-propanol kommen insbesondere 1,3-Dichlor-2-propanol und 1,3-Dibrom-2-propanol in Betracht.

Die Umsetzungen der 1,3,4-Oxadiazolylphenole zur Herstellung der Ausgangsverbindungen der Formel II werden zweckmäßigerweise bei Temperaturen von 0 bis 120° C und unter Normaldruck oder in einem geschlossenen Gefäß unter erhöhten Drucken durchgeführt. Die Umsetzungen werden zweckmäßigerweise in einem inerten Verdünnungs- oder Lösungsmittel, z. B. einem niederen aliphatischen Keton, wie Aceton, Methyläthylketon oder Methylisobutylketon, einem niederen Alkohol mit 1 bis 4 C-Atomen, wie Methanol, Äthanol, Propanol oder Butanol, einem gesättigten aliphatischen oder cyclischen Äther, wie Dialkyläther, Tetrahydrofuran oder Dioxan, einem Dialkylformamid, wie Dimethylformamid oder Diäthylformamid oder Hexamethylphosphortriamid oder mit überschüssigem Alkylierungsmittel als Verdünnungs- oder Lösungsmittel durchgeführt.

Bevorzugt werden die Umsetzungen in Gegenwart einer Base als säurebindendes Mittel vorgenommen. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin oder niedere Trialkylamine, wie Trimethyl- oder Triäthylamin. Dabei können die Basen im Verhältnis zum eingesetzten Alkylierungsmittel in katalytischer Menge oder in stöchiometrischer Menge bzw. in geringem Überschuß verwendet werden.

Bevorzugt werden die 1,3,4-Oxadiazolylphenole mit Epibromhydrin oder 1,2-Dibrompropanol-2 in einem Lösungsmittelgemisch aus einem Äther und einem polaren aprotischen Lösungsmittel, insbesondere Tetrahydrofuran und Hexamethylphosphortriamid, bei Temperaturen zwischen 0 und 50° C umgesetzt.

Weiterhin sei erwähnt, daß die Ausgangsverbindungen der Formel II durch einfache Säure-Base-Reaktion ineinander umgewandelt werden können. So läßt sich ein 2,3-Epoxypropoxyphenyl-1,3,4-oxadiazol mit der entsprechenden Halogenwasserstoffsäure in ein 2-Hydroxy-3-halogenpropoxyphenyl-1,3,4-oxadiazol überführen, wobei als Verdünnungs- oder Lösungsmittel neben an sich üblichen Solventien vorzugsweise aliphatische oder cyclische Äther, wie Diäthyläther, Tetrahydrofuran oder Dioxan, oder ein niederer Alkohol, wie Methanol, Äthanol oder Propanol, verwendet werden. Andererseits können die 2-Hydroxy-3-halogenpropoxyphenyl-1,3,4-oxadiazole mit einer Base, wie einem Alkalimetall-hydroxid, -carbonat, -hydrogencarbonat, -alkoholat oder -hydrid, einem tertiären organischen Amin, wie Pyridin, oder einem tertiären aliphatischen Amin, insbesondere Trimethylamin oder Triäthylamin, oder auch Piperidin in 2,3-Epoxypropoxyphenyl-1,3,4-oxadiazole umgewandelt werden. Diese Reaktionen können bei Raumtemperatur durchgeführt werden oder durch Wärmezufuhr, z. B. durch Erwärmen auf 60 bis 120° C, beschleunigt oder abgeschlossen werden.

Die Reaktion kann unter Atmosphärendruck oder in einem geschlossenen Gefäß unter erhöhtem Druck gegebenenfalls bei gleichzeitigem Erwärmen durchgeführt werden. Die Ausgangsstoffe für diese Umwandlung können zuvor isoliert oder im Reaktionsgemisch erzeugt und ohne weitere Isolierung und Reinigung unmittelbar weiterverarbeitet werden.

Die erfindungsgemäßen Verbindungen der Formel (I) weisen am Kohlenstoffatom 2 der aliphatischen Seitenkette ein Chiralitätszentrum auf und werden als Racemate erhalten, die durch bekannte Methoden, beispielsweise durch Bildung diastereomerer Salze mit optisch aktiven Hilfssäuren, wie Dibenzoylweinsäure, Campfer-10-sulfonsäure, Ditoluylweinsäure oder 3-Brom-campfer-8-sulfonsäure, in die optisch aktiven Antipoden getrennt werden können.

Gegebenenfalls werden die erhaltenen erfindungsgemäßen Verbindungen in das Säureadditionssalz einer physioligisch verträglichen Säure überführt. Als übliche physiologisch verträgliche anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzeneimittelforschung Band 10, Seiten 224 bis 225, Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen

Lösungsmittel, beispielsweise einem niederen Alkohol wie Methanol, Äthanol oder Propanol oder einem niederen Keton wie Aceton, Methyläthylketon oder Methylisobutylketon oder einem Äther wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Phenylpiperazinyl-Derivate der allgemeinen Formel (I) durch Auflösen der freien Base der allgemeinen Formel (I) in einer wäßrigen Säurelösung hergestellt werden.

Die erfindungsgemäßen Verbindungen und ihre physiologisch verträglichen Säureadditionssalze sind als Pharmaka mit blutdrucksenkender Wirkung zur Behandlung der Hypertonie geeignet.

Die blutdrucksenkende Wirkung wurde an Ratten getestet. Als Vergleichssubstanz diente das Antihypertonikum Urapidil (6-{3-[4-(2-Methoxyphenyl)-1-piperazinyl]-propylamino}-1,3-dimethyluracil).

Zur Untersuchung wurden männliche Sprague-Dawley-Ratten im Gewicht von 220-280 g in Urethan-Narkose (1,78 g/kg i. p.) verwendet. Der Blutdruck wurde blutig in der Aorta carotis gemessen. Die Substanzapplikation erfolgte intravenös (i. v.) in die Vena jugularis.

Als ED 20% werden die Dosen (mg/kg) ermittelt, welche eine 20%ige Blutdrucksenkung bewirken.

Außer der blutdrucksenkenden Wirkung wird die akute Toxizität (LD 50, mg/kg) an Gruppen von je 10 weiblichen NMRI-Mäusen, Gewicht 22 bis 27 g, bei intraperitonealer Applikation ermittelt.

Die therapeutische Breite ist der Quotient aus der LD 50 und der ED 20%.

Die erfindungsgemäßen Verbindungen besitzen starke blutdrucksenkende Wirkung. Aus der Tabelle 1 geht hervor, daß ihre Wirksamkeit 2,5 bis 22,5 mal höher ist als die der Vergleichssubstanz Urapidil. Die therapeutische Breite ist bis zu 13mal größer als die des Urapidil. Gegenüber den Verbindungen der DE-OS 28 11 638 sind sie nicht als $\beta$-sympatholytisch, sondern als selektiv blutdrucksenkend bzw. antihypertensiv zu klassifizieren.

Tabelle 1

| Verbindung Beispiel Nr. | Blutdrucksenkende Wirkung[1]) | | Toxizität[3]) | Therapeutische Breite[4]) |
|---|---|---|---|---|
| | ED 20% | R. W.[2]) | LD 50 | |
| 2 | 0,0221 | 8,55 | 56,2 | 2540 |
| 7 | 0,0754 | 2,51 | 215 | 2850 |
| 9 | 0,00840 | 22,50 | 215 | 25600 |
| 10 | 0,0333 | 5,68 | 224 | 6730 |
| 12 | 0,0195 | 9,69 | 162 | 8300 |
| 16 | 0,0716 | 2,64 | 178 | 2490 |
| Urapidil | 0,189 | 1,00 | 362 | 1920 |

[1]) Ratte. Urethan-Narkose. Appl.: i. v.
[2]) Relative Wirksamkeit. Urapidil = 1,00.
[3]) Maus. Appl. i. p.
[4]) $\frac{\text{LD 50}}{\text{ED 20\%}}$

Gegenstand der vorliegenden Erfindung sind demnach auch therapeutische Mittel oder Zubereitungen, die neben üblichen Träger- und Verdünnungsmitteln eine Verbindung der Formel I oder deren physiologisch verträgliches Säureadditionssalz als Wirkstoff enthalten, sowie die Verwendung der neuen Verbindungen bei der Behandlung der Hypertonie.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln verarbeitet werden (vgl. L. G. Goodman, A. Gilman, The Pharmacological Basis of Therapeutics). Die so erhaltenen Präparate enthalten den Wirkstoff normalerweise in einer Menge von 0,001 und 99 Gew.-%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Es kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmittel, wie Maisstärke oder Alginsäure, Bindemittel wie Stärke oder Gelatine, Gleitmittel wie Magnesiumstearat oder Talk und/oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Vanillin oder Orangenextrakt, enthalten. Sie können außerdem Suspendierstoffe, die Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyäthylenglykol bzw. deren Derivaten herstellen.

Die Dosierung der erfindungsgemäßen Verbindungen hängt vom Alter, Zustand und Gewicht des Patienten sowie von der Applikationsart ab. In der Regel beträgt die tägliche Wirkstoffdosis 5 bis 100, vorzugsweise 10 bis 80 mg.

Die vorliegende Erfindung wird durch die nachfolgenden Ausführungsbeispiele näher erläutert.

## I. Herstellung von Ausgangsverbindungen

I. 2-(1,3,4-Oxadiazolyl-2)-phenol

90 g (0,6 Mol) Salicylsäurehydrazid und 355,2 g (2,4 Mol) Orthoameisensäureethylester werden für 22 Stunden zum Rückfluß erhitzt. Überschüssiger Orthoameisensäureester wird abdestilliert und der feste Rückstand aus Äthanol umkristallisiert. 62,9 g farblose Kristalle (64,7% d. Th.), Fp. 112—113°C.

II. 2-(5-Methyl-1,3,4-oxadiazolyl-2)-phenol

90 g (0,6 Mol) Salicylsäurehydrazid und 388,8 g (2,4 Mol) Orthoessigsäuretriäthylester werden in 500 ml n-Propanol für 84 Stunden zum Rückfluß erhitzt. Nach dem Abkühlen wird der Niederschlag abgesaugt und aus Toluol/Petroläther umkristallisiert. 47 g farblose Kristalle (26,7% d. Th.), Fp. 74—76°C.

III. 3-(1,3,4-Oxadiazolyl-2)-phenol

Wird analog Beispiel I hergestellt. 83 g farblose Kristalle (86% d. Th.), Fp. 215-216°C.

IV. 3-(5-Methyl-1,3,4-oxadiazolyl-2)-phenol

Wird analog Beispiel II hergestellt. 94 g farblose Kristalle (89% d. Th.), Fp. 174—175°C.

V. 4-(1,3,4-Oxadiazolyl-2)-phenol

Wird analog Beispiel I erhalten. 87 g farblose Kristalle (89,5% d. Th.), Fp. 215°C.
Wird analog Beispiel II hergestellt.

VI. para-(5-Methyl-1,3,4-oxadiazolyl-2)-phenol

101 g farblose Kristalle (96% d. Th.), Fp. 232°C.

VII. 2,3-Epoxypropoxy-2-(1,3,4-oxadiazolyl-2)-benzol

Analog Beispiel VIII werden 4,3 g (55% d. Th.) hellgelbes Öl erhalten, das ein den Erwartungen entsprechendes NMR-Spektrum zeigt:

$^1$H-NMR ($CDCl_3$): $\delta = 8{,}5$ (1 Hs, Oxadiazol-Proton):
$\delta = 7{,}0-8{,}0$ (4 Hm, aromatische Protonen)
$\delta = 4{,}2$ (2 Hm), $\delta = 3{,}4$ (1 Hm und $\delta = 2{,}85$ (2 Hd) für die Epoxypropylprotonen.

VIII. 2,3-Epoxypropoxy-2-(5-methyl-1,3,4-oxadiazolyl-2)-benzol

1,6 g Natriumhydrid als 55%ige Suspension in Paraffinöl (0,036 Mol) werden in 70 ml wasserfreiem Tetrahydrofuran vorgelegt und tropfenweise mit 6,3 g (0,036 Mol) ortho-(5-Methyl-1,3,4-oxadiazolyl-2)-phenol gelöst in 50 ml Tetrahydrofuran versetzt. Anschließend werden 5 g (0,036 Mol) Epibromhydrin zugetropft und 10 ml Hexamethylphosphorsäuretriamid zum Reaktionsgemisch gegeben und für 32 Stunden bei Raumtemperatur gerührt. Das Reaktionsgemisch wird auf 500 ml wäßrige Natriumchloridlösung gegeben und mit Diäthyläther mehrfach ausgeschüttelt. Die vereinigten Ätherauszüge werden über wasserfreiem Natriumsulfat getrocknet und am Rotationsverdampfer zu einem gelben Öl eingeengt.

Mit Toluol/Hexan werden Kristalle erhalten. 5,5 g (66% d. Th.) farblose Kristalle, Fp. 38—40° C.

$C_{12}H_{12}N_2O_3$ (232)

| | | | |
|---|---|---|---|
| berechnet | C: 62,1 | H: 5,2 | N: 12,1 |
| gefunden | C: 61,6 | H: 5,3 | N: 12,1 |

IX. 2,3-Epoxypropoxy-3(1,3,4-oxadiazolyl-2)-benzol

Analog Beispiel VIII werden 4,2 g (53% d. Th.) farblose Kristalle erhalten, Fp. 80—82° C.

X. 2,3-Epoxypropoxy-3-(5-methyl-1,3,4-oxadiazolyl-2)-benzol

Analog Beispiel VIII werden 5,7 g (68% d. Th.) farblose Kristalle erhalten, Fp. 56° C.

XI. 2,3-Epoxypropoxy-4-(1,3,4-oxadiazolyl-2)-benzol

Analog Beispiel VIII werden 5,4 g (69% d. Th.) farblose Kristalle erhalten, Fp. 81—82° C.

$C_{11}H_{10}O_3N_2$ (218)

| | | | |
|---|---|---|---|
| berechnet | C: 60,6 | H: 4,6 | N: 12,8 |
| gefunden | C: 61,2 | H: 5,2 | N: 12,4 |

XII. 2,3-Epoxypropoxy-4-(5-methyl-1,3,4-oxadiazolyl-2)-benzol

Analog Beispiel VIII werden 5,9 g (71% d. Th.) farblose Kristalle erhalten, Fp. 65—67° C.

## II. Herstellung von erfindungsgemäßen Verbindungen

### Beispiel 1

1-[2-(1,3,4-Oxadiazolyl-2)-phenoxy]-3-[4-(2-methoxyphenyl)-piperazinyl-1]-propanol-2

7,3 g (0,033 Mol) 2,3-Epoxypropoxy-2-(1,3,4-oxadiazolyl-2)-benzol aus Beispiel VII und 6,4 g (0,033 Mol) 2-Methoxyphenylpiperazin werden in 50 ml Äthanol für 17 Stunden zum Rückfluß erhitzt. Das Lösungsmittel wird am Rotationsverdampfer abgezogen und der ölige Rückstand in Methylenchlorid aufgenommen. Die Lösung wird 3mal mit je 50 ml Wasser gewaschen, über wasserfreiem Natriumsulfat getrocknet, abfiltriert und eingeengt. Man erhält 12,6 g (93% d. Th.) eines hellgelben Öls.

Aus Äthanol/Diäthyläther wird mit ätherischer Salzsäure das Hydrochlorid gefällt (hygroskopisch), mit Isopropanol gewaschen und bei 80° C im Vakuum getrocknet.

4,9 g (29%) farblose Kristalle, Fp. 129—130° C

$C_{22}H_{26}N_4O_4 \cdot 1{,}5\ HCl \cdot H_2O$ (482)

| | | | | | |
|---|---|---|---|---|---|
| berechnet | C: 54,7 | H: 6,1 | Cl: 10,9 | N: 11,6 | O: 16,6 |
| gefunden | C: 54,8 | H: 6,7 | Cl: 10,0 | N: 11,8 | O: 16,5 |

### Beispiel 2

1-[2-(5-Methyl-1,3,4-oxadiazolyl-2)-phenoxy]-3-[2-methoxyphenyl)-piperazinyl-1]-propanol-2

wird analog Beispiel 1 erhalten.

3,1 g (20% d. Th.) farblose Kristalle, Fp. 248—249° C.

$C_{23}H_{28}N_4O_4 \cdot HCl$ (461)

| | | | | | |
|---|---|---|---|---|---|
| berechnet | C: 59,9 | H: 6,3 | Cl: 7,6 | N: 12,2 | O: 13,9 |
| gefunden | C: 59,0 | H: 6,2 | Cl: 7,7 | N: 12,1 | O: 14,7 |

Beispiel 3

1-[2-(1,3,4-Oxadiazolyl-2)-phenoxy]-3-[4-methoxyphenyl)-piperazinyl-1]-propanol-2

8,1 g (0,037 Mol) 2,3-Epoxypropoxy-2(1,3,4-oxadiazolyl-2)-benzol aus Beispiel VII werden mit 7,1 g (0,037 Mol) 3-Methoxyphenylpiperazin in 100 ml Isobutanol für 18 Stunden zum Rückfluß erhitzt. Aufarbeitung erfolgt analog Beispiel 1. 11,1 g (62% d. Th.) farblose Kristalle, Fp. 126—128° C.

$C_{22}H_{26}O_4N_4 \cdot 2\ HCl$ (438)

| | | | | |
|---|---|---|---|---|
| berechnet | C: 54,7 | H: 5,8 | Cl: 14,7 | N: 11,6 |
| gefunden | C: 54,4 | H: 6,2 | Cl: 12,2 | N: 11,9 |

Beispiel 4

1-[2-(5-Methyl-1,3,4-oxadiazolyl-2)-phenoxy]-3-[4-(3-methoxyphenyl)-piperazinyl-1]-propanol-2

wird analog Beispiel 1 erhalten. 11,6 g (66% d. Th.) farblose Kristalle, Fp. 201—202° C.

$C_{23}H_{28}N_4O_4 \cdot 2\ HCl \cdot 2\ H_2O$ (533)

| | | | |
|---|---|---|---|
| berechnet | C: 51,8 | H: 6,4 | N: 10,5 |
| gefunden | C: 52,6 | H: 6,4 | N: 10,6 |

Beispiel 5

1-[2-(5-Methyl-1,3,4-oxadiazolyl-2)-phenoxy]-3-[4-(3-chlorphenyl)-piperazinyl-1]-propanol-2

wird analog Beispiel 3 erhalten. 6,6 g (40% d. Th.) farblose Kristalle, Fp. 115—117° C.

$C_{22}H_{25}ClN_4O_3 \cdot 2\ HCl$ (501)

| | | | |
|---|---|---|---|
| berechnet | C: 52,7 | H: 5,4 | N: 11,2 |
| gefunden | C: 52,4 | H: 5,5 | N: 11,1 |

Beispiel 6

1-[2-(5-Methyl-1,3,4-oxadiazolyl-2)-phenoxy]-3-[4-(4-fluorphenyl)-piperazinyl-1]-propanol-2

wird analog Beispiel 1 in Isopropanol als Lösungsmittel erhalten. 6,6 g (41% d. Th.) farblose Kristalle, Fp. 212-213° C.

$C_{22}H_{25}FN_4O_3 \cdot 2\ HCl$ (485)

| | | | | |
|---|---|---|---|---|
| berechnet | C: 54,4 | H: 5,4 | N: 11,5 | Cl: 14,6 |
| gefunden | C: 54,1 | H: 5,8 | N: 11,4 | Cl: 14,3 |

Beispiel 7

1-[3-(1,3,4-Oxadiazolyl-2)-phenoxy]-3-[4-(2-methoxyphenyl)-piperazinyl-1]-propanol-2

wird analog Beispiel 1 in Äthanol als Lösungsmittel erhalten. 3,6 g (22% d. Th.) farblose Kristalle, Fp. 208—210° C.

$C_{22}H_{26}N_4O_4 \cdot 1{,}5\ HCl \cdot 1{,}5\ H_2O$ (492)

| | | | |
|---|---|---|---|
| berechnet | C: 53,7 | H: 6,2 | N: 11,4 |
| gefunden | C: 53,2 | H: 6,5 | N: 11,0 |

Beispiel 8

1-[3-(1,3,4-Oxadiazolyl-2)-phenoxy]-3-[4-(3-methoxyphenyl)-piperazinyl-1]-propanol-2

wird analog Beispiel 1 in Isopropanol als Lösungsmittel erhalten. 6,6 g (40% d. Th.) farblose Kristalle, Fp. 208—209° C.

$C_{22}H_{26}N_4O_4 \cdot 1,5\ HCl \cdot 1,5\ H_2O$ (492)

| | | | |
|---|---|---|---|
| berechnet | C: 53,7 | H: 6,2 | N: 11,4 |
| gefunden | C: 53,4 | H: 6,4 | N: 11,3 |

Beispiel 9

1-[3-(1,3,4-Oxadiazolyl-2)-phenoxy]-3-[4-(2-äthoxyphenyl)-piperazinyl-1]-propanol-2

wird analog Beispiel 1 in Isopropanol als Lösungsmittel erhalten. 13,4 g (79% d. Th.) farblose Kristalle, Fp. 211—212° C.

$C_{23}H_{28}N_4O_4 \cdot 2\ HCl$ (497)

| | | | |
|---|---|---|---|
| berechnet. | C: 55,4 | H: 6,0 | N: 11,3 |
| gefunden | C: 54,7 | H: 6,3 | N: 11,0 |

Beispiel 10

1-[3-(5-Methyl-1,3,4-oxadiazolyl-2)-phenoxy]-3-[4-(2-methoxyphenyl)-piperazinyl-1]-propanol-2

wird analog Beispiel 1 in Isopropanol als Lösungsmittel erhalten. 11,2 g (68,6% d. Th.) farblose Kristalle, Fp. 205—206° C.

$C_{23}H_{28}N_4O_4 \cdot 2\ HCl$ (497)

| | | | | |
|---|---|---|---|---|
| berechnet | C: 55,5 | H: 6,1 | N: 11,3 | Cl: 14,3 |
| gefunden | C: 55,4 | H: 6,2 | N: 11,4 | Cl: 14,0 |

Beispiel 11

1-[3-(5-Methyl-1,3,4-oxadiazolyl-2)-phenoxy]-3-[4-(3-methoxyphenyl)-piperazinyl-1]-propanol-2

wird analog Beispiel 1 in Äthanol erhalten, das freie Piperzinderivat kristallisiert aus der Reaktionslösung aus und wird abgesaugt. 10,1 g (72% d. Th.) farblose Kristalle, Fp. 125—127° C.

$C_{23}H_{28}N_4O_4$ (424)

| | | | |
|---|---|---|---|
| berechnet | C: 65,1 | H: 6,6 | N: 13,2 |
| gefunden | C: 64,9 | H: 6,6 | N: 13,3 |

Beispiel 12

1-[3-(5-Methyl-1,3,4-oxadiazolyl-2)-phenoxy]-3-[4-(2-äthoxyphenyl)-piperazinyl-1]-propanol-2

wird analog Beispiel 1 in Isopropanol als Lösungsmittel erhalten. 14,6 g (87% d. Th.) farblose Kristalle, Fp. 200—202° C.

$C_{24}H_{30}N_4O_4 \cdot 2\ HCl$ (511)

| | | | | |
|---|---|---|---|---|
| berechnet | C: 56,4 | H: 6,3 | N: 11,0 | Cl: 13,9 |
| gefunden | C: 56,3 | H: 6,3 | N: 11,1 | Cl: 13,8 |

Beispiel 13

1-[4-(1,3,4-Oxadiazolyl-2)-phenoxy]-3-[4-(3-methoxyphenyl)-piperazinyl-1)-propanol-2

wird analog Beispiel 1 in Isopropanol als Lösungsmittel erhalten, wobei das freie Piperazinderivat direkt aus der Reaktionslösung auskristallisiert und abgesaugt wird. 12 g (98% d. Th.) farblose Kristalle, Fp. 149—150° C.

$C_{22}H_{26}N_4O_4$ (410)

| | | | |
|---|---|---|---|
| berechnet | C: 64,4 | H: 6,4 | N: 13,6 |
| gefunden | C: 64,1 | H: 6,5 | N: 13,4 |

Beispiel 14

1-[4-(1,3,4-Oxadiazolyl-2-)-phenoxy]-3-[4-(4-fluorphenyl)-piperazinyl-1]-propanol-2

wird analog Beispiel 1 in Isopropanol als Lösungsmittel erhalten, wobei das freie Piperazinderivat direkt aus der Reaktionslösung auskristallisiert und abgesaugt wird. 11,1 g (81% d. Th.) farblose Kristalle, Fp. 226—228° C.

$C_{21}H_{23}FN_4O_3 \cdot H_2O$ (416)

| | | | |
|---|---|---|---|
| berechnet | C: 60,6 | H: 6,1 | N: 13,4 |
| gefunden | C: 60,8 | H 6,4 | N 12,4 |

Darüber hinaus werden die folgenden erfindungsgemäßen Verbindungen hergestellt:

Beispiel 15

1-[3-(5-Methyl-1,3,4-oxadiazolyl-2)-phenoxy]-3-[4-(3-chlorphenyl)-piperazinyl-1]-propanol-2 (· 2 HCl), Fp. 172—173° C

Beispiel 16

1-[3-(5-Methyl-1,3,4-oxadiazolyl-2)-phenoxy]-3-[4-(2-chlorphenyl)-piperazinyl-1]-propanol-2 ( · 2 HCl · $H_2O$), Fp. 216—218° C

Beispiel 17

1-[3-(1,3,4-Oxadiazolyl-2)-phenoxy]-3-[4-(2-chlorphenyl)-piperazinyl-1]-propanol-2, Fp. 130—132° C

Beispiel 18

1-[3-(1,3,4-Oxadiazolyl-2)-phenoxy]-3-[4-(2,6-dimethylphenyl)-piperazinyl-1]-propanol-2 ( · HCl), Fp. 222—223° C

Beispiel 19

1-[3-(5-Methyl-1,3,4-oxadiazolyl-2)-phenoxy]-3-[4-(2,6-dimethylphenyl)-piperazinyl-1-]-propanol-2 ( · HCl), Fp. 187—188° C

Beispiel 20

1-[3-(5-Methyl-1,3,4-oxadiazolyl-2)-phenoxy]-3-[4-(2,4-dimethoxyphenyl)-piperazinyl-1]-propanol-2 ( · 2,5 HCl · 0,5 $H_2O$), Fp. 195—198° C

Beispiel 21

1-[3-(1,3,4-Oxadiazolyl-2)-phenoxy]-3-[4-(2,4-dimethoxyphenyl)-piperazinyl-1]-propanol-2, Fp. 144—147° C

Beispiel 22

1-[3-(1,3,4-Oxadiazolyl-2)-phenoxy]-3-(4-phenyl-piperazinyl-1)-propanol-2, Fp. 136—138° C

Beispiel 23

1-[3-(5-Methyl-1,3,4-oxadiazolyl-2)-phenoxy]-3-[4-phenylpiperazinyl-1]-propanol-2

Beispiel 24

1-[3-(5-Methyl-1,3,4-oxadiazolyl-2)-phenoxy]-3-[4-(4-methoxyphenyl)-piperazinyl-1]-propanol-2, Fp. 166—168° C.

Beispiel 25

1-[4-(1,3,4-Oxadiazolyl-2)-phenoxy]-3-[4-(2-methoxyphenyl)-piperazinyl-1]-propanol-2 ( · 2 HCl · $H_2O$), Fp. 225° C

Beispiel 26

1-[4-(1,3,4-Oxadiazolyl-2)-phenoxy]-3-[4-(2-chlorphenyl)-piperazinyl-1]-propanol-2 ( · 2 HCl), Fp. 249—251° C

Beispiel 27

1-[4-(5-Methyl-1,3,4-oxadiazolyl-2)-phenoxy]-3-[4-(2-methoxyphenyl)-piperazinyl-1]-propanol-2 ( · Oxalat · $H_2O$), Fp. 162—163° C

Beispiel 28

1-[4-(5-Methyl-1,3,4-oxadiazolyl-2)-phenoxy]-3-[4-(3-methoxyphenyl)-piperazinyl-1]-propanol-2( · 2,5 HCl · $H_2O$), Fp. 230—233° C

Beispiel 29

1-[4-(1,3,4-Oxadiazolyl-2)-phenoxy]-3-[4-(2,4-dimethoxyphenyl)-piperazinyl-1]-propanol-2, Fp. 161° C.

III. Formulierungsbeispiele, die in üblicher Weise hergestellt werden

1. Tabletten:

| | | |
|---|---|---|
| a) | Ein Wirkstoff der Formel I | 5 mg |
| | Lactose | 200 mg |
| | Methylcellulose | 15 mg |
| | Maisstärke | 50 mg |
| | Talkum | 11 mg |
| | Magnesiumstearat | 4 mg |
| b) | Ein Wirkstoff der Formel I | 20 mg |
| | Lactose | 178 mg |
| | Avicel® | 80 mg |
| | Polywachs 6000 | 20 mg |
| | Magnesiumstearat | 2 mg |
| c) | Ein Wirkstoff der Formel I | 50 mg |
| | Polyvinylpyrrolidon (mittl. M. G. 25 000) | 170 mg |
| | Polyäthylenglykol (mittl. M. G. 4000) | 14 mg |
| | Hydroxypropylmethylcellulose | 40 mg |
| | Talkum | 4 mg |
| | Magnesiumstearat | 2 mg |

zu c) Der Wirkstoff wird mit Polyvinylpyrrolidon in 10%iger wäßriger Lösung befeuchtet, durch ein Sieb mit der lichten Maschenweite 1,0 mm getrieben und bei 50° C getrocknet. Dieses

Granulat wird mit Polyäthylenglykol (mittl. M. G. 4000), Hydroxypropylmethylcellulose, Talkum und Magnesiumstearat vermischt und zu Tabletten à 280 mg verpreßt.

2. Beispiel für Dragees

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50° C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum bestehen.

3. Kapselformulierung

| | |
|---|---|
| Ein Wirkstoff der Formel I | 5 mg |
| Magnesiumstearat | 2,0 mg |
| Milchzucker | 19,3 mg |

4. Injektionslösung

| | |
|---|---|
| Ein Wirkstoff der Formel I | 10 mg |
| Natriumchlorid | 9 mg |
| destilliertes Wasser, q. s. auf 1,0 ml | |

**Patentansprüche**

1. Verbindungen der allgemeinen Formel I,

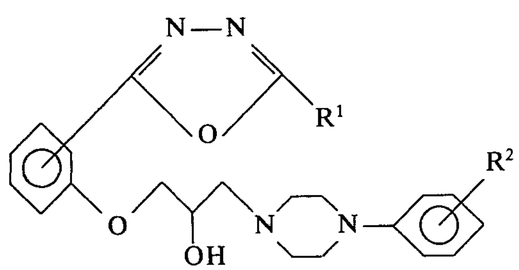

(I)

in der $R^1$ Wasserstoff oder Methyl und $R^2$ Wasserstoff, ein Fluoratom, Chloratom, eine Methyl-, Methoxy- oder Ethoxygruppe, wobei $R^2$ als Substituent des Phenylringes ein- oder zweifach vorhanden sein kann, bedeuten, und ihre physiologisch verträglichen Säureadditionssalze.

2. 1-[3-(5-Methyl-1,3,4-oxadiazolyl-2-phenoxy]-3-[4-(2-methoxyphenyl)-piperazinyl-1]-propanol-2 und dessen physiologisch verträgliche Säureadditionssalze.

3. Verfahren zur Herstellung von Verbindungen der allgemeinen Formel I nach Anspruch 1, dadurch gekennzeichnet, daß man ein 1,3,4-Oxadiazolyl-2-phenylderivat der allgemeinen Formel II

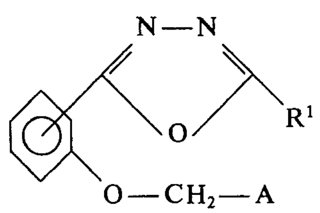

(II)

in der $R^1$ die für Formel I angegebenen Bedeutungen hat und in der A den Rest

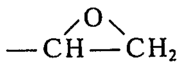 oder 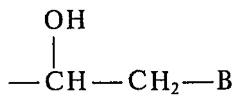

wobei B für eine nukleofuge Abgangsgruppe steht, bedeutet, mit einem Phenylpiperazin der allgemeinen Formel III

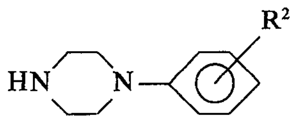

(III)

in der $R^2$ die für Formel I angegebenen Bedeutungen hat, zweckmäßigerweise in einem Lösungsmittel und gegebenenfalls in Gegenwart eines säurebindenden Mittels in an sich bekannter Weise umsetzt und die erhaltene Verbindung gegebenenfalls in das Säureadditionssalz einer physiologisch verträglichen Säure überführt.

4. Therapeutisches Mittel, gekennzeichnet durch einen Gehalt an einer Verbindung der Formel I oder ihres physiologisch verträglichen Säureadditionssalzes als Wirkstoff neben üblichen Trägerstoffen und Verdünnungsmitteln.

5. Therapeutisches Mittel nach Anspruch 4, gekennzeichnet durch einen Gehalt an 1-[3-(5-Methyl-1,3,4-oxadiazolyl-2-phenoxy]-3-[4-(2-methoxyphenyl)-piperazinyl-1]-propanol-2 oder dessen physiologisch verträgliche Säureadditionssalze.

## Claims

1. A compound of the general formula I

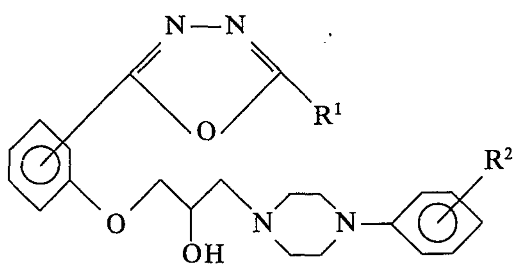

(I)

where $R^1$ is hydrogen or methyl and $R^2$ is hydrogen, fluorine, chlorine, methyl, methoxy or ethoxy, and where the phenyl ring may contain one or two substituents $R^2$, and its physiologically tolerated addition salts with acids.

2. 1-[3-(5-Methyl-1,3,4-oxadiazol-2-yl)-phenoxy]-3-[4-(2-methoxyphenyl)-piperazin-1-yl]-propan-2-ol and its physiologically tolerated addition salts with acids.

3. A process for the preparation of a compound of the general formula I as claimed in claim 1, wherein a 1,3,4-oxadiazol-2-yl-phenyl derivative of the general formula II

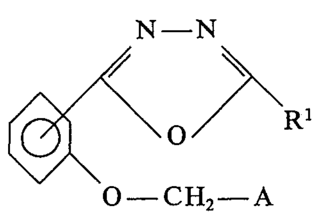

(II)

where $R^1$ has the meanings given for formula I and A is

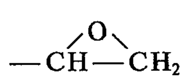 or 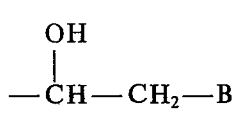

B being a nucleofugic leaving group, is reacted, in a conventional manner, with a phenylpiperazine of the general formula III

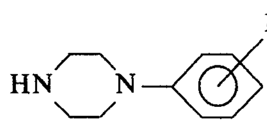

(III)

where $R^2$ has the meanings given for formula I, advantageously in a solvent and in the presence or absence of an acid acceptor, and, if desired, the compound obtained is converted into the addition salt with a physiologically tolerated acid.

4. A therapeutic agent containing a compound of the formula I, or a physilogically toerated addition salt thereof with an acid, as the active compound, in addition to conventional carriers and diluents.

5. A therapeutic agent as claimed in claim 4, containing 1-[3-(5-Methyl-1,3,4-oxadiazol-2-yl)-phenoxy]-3-[4-(2-methoxyphenyl)-piperazin-1-yl]-propan-2-ol or a physiologically tolerated addition salt thereof with an acid.

**Revendications**

1. Composés de formule générale I

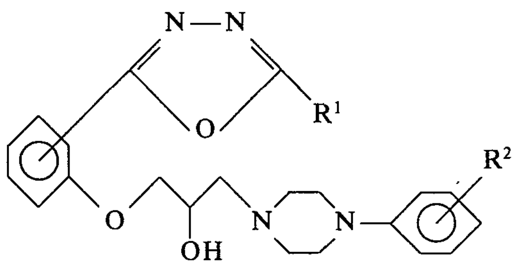

(I)

dans laquelle $R^1$ représente hydrogène ou méthyle et $R^2$, hydrogène, un atome de fluor, un atome de chlore, un groupe méthyle, méthoxy, ou éthoxy, $R^2$ pouvant être présent une fois ou deux comme substituant du noyau phényle et leurs sels d'addition d'acide acceptables physiologiquement.

2. 1-[3-(5-méthyl-1,3,4-oxadiazolyl-2-phénoxy]-3-[4-(2-méthoxyphényl)-pipérazinyl-1]-propanol-2 et ses sels d'addition d'acide acceptables physiologiquement.

3. Procédé de préparation de composés de formule générale I, selon la revendication 1, caractérisé par le fait que, de manière connue en soi, de préférence dans un solvant et éventuellement en présence d'un agent liant d'acide, on fait réagir un dérive de 1,3,4-oxadiazolyl-2-phényle de formule générale II

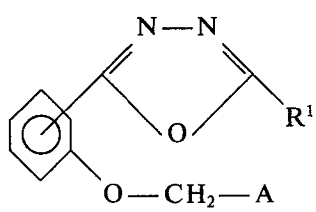

(II)

dans laquelle $R^1$ a les significations indiquées pour la formula I et où A représente le reste

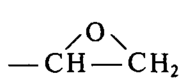 ou 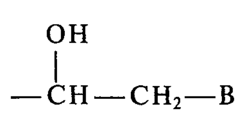

B étant un groupe de départ nucléofuge, avec une phénylpipérazine de formule générale III

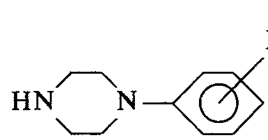

(III)

dans laquelle $R^2$ a les significations données pour la formule I, et l'on transforme éventuellement le composé obtenu en le sel d'addition d'un acide acceptable physiologiquement.

4. Agent thérapeutique, caractérisé par une teneur en un composé de formule I ou son sel d'addition d'acide acceptable physiologiquement, en tant que principe actif, outre les véhicules et diluants

usuels.

5. Agent thérapeutique selon la revendication 4, caractérisé par une teneur en 1-[3-(5-méthyl-1,3,4-oxadiazolyl-2-phénoxy]-3-[4-(2-méthoxyphényl)-pipérazinyl-1]-propanol-2 ou ses sels d'addition d'acide acceptables physiologiquement.